(19) Europäisches Patentamt
European Patent Office
Office européen des brevets

(11) **EP 3 486 326 A1**

(12) **EUROPEAN PATENT APPLICATION**

(43) Date of publication:
**22.05.2019 Bulletin 2019/21**

(21) Application number: **17202833.4**

(22) Date of filing: **21.11.2017**

(51) Int Cl.:
*C12P 19/26* [(2006.01)]  *C07H 13/02* [(2006.01)]
*C07H 1/06* [(2006.01)]  *A23L 33/125* [(2016.01)]

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Jennewein Biotechnologie GmbH
53619 Rheinbreitbach (DE)**

(72) Inventors:
• **Jennewein, Stefan
53604 Bad Honnef (DE)**
• **Helfrich, Markus
53557 Bad Hönningen (DE)**

(54) **METHOD FOR THE PURIFICATION OF N-ACETYLNEURAMINIC ACID FROM A FERMENTATION BROTH**

(57) Disclosed is a method for the purification of *N*-acetylneuraminic acid (Neu5Ac) from fermentation broth, a composition and a spray-dried, GMO-free powder containing Neu5Ac from fermentation broth, and the use of said Neu5Ac for producing a nutritional composition such as infant formula.

**FIG. 1**

Printed by Jouve, 75001 PARIS (FR)

**Description**

The present invention concerns the purification of sialic acids. More specifically, the present invention concerns the purification of *N*-acetylneuraminic acid from a fermentation broth. The present invention also relates to the *N*-acetylneuraminic acid obtained by said method, to the use of said *N*-acetylneuraminic acid for the manufacture of nutritional compositions, and to nutritional compositions containing said *N*-acetylneuraminic acid.

**Background**

Sialic acids (Sia) are a family of negatively charged monosaccharides with a nine-carbon backbone. More than 50 forms of these α-keto acids have been found in nature. The most abundant sialic acid appears to be *N*-acetylneuraminic acid (NANA, NeuNAc, Neu5Ac).

Sialic acids are present as the terminal saccharides of the glycans present in cell-surface glycoconjugates (glycoproteins and glycolipids) in vertebrates and higher invertebrates. Sialic acids are also components of the lipopolysaccharides and capsular polysaccharides of pathogenic bacteria including *Escherichia coli* K1, *Haemophilus influenzae, Haemophilus ducreyi, Pasteurella multocida, Neisseria gonorrhoeae, Neisseria meningitidis, Campylobacter jejuni* and *Streptococcus agalactiae.*

Sialic acids play important roles in many physiological and pathophysiological processes including the development of the embryonic nervous system, metastasis, the regulation of immune responses, and infections with bacteria or viruses. Sialic acids are an essential component of brain gangliosides and of the polysialic acid chains that modify neural cell adhesion molecules (NCAMs) that facilitate cell-to-cell interactions, neuronal outgrowth, the modification of synaptic connectivity and memory formation. In piglets, a diet rich in sialic acids increases the level of brain sialic acids and the expression of two learning-related genes. Accordingly, the diet also enhances learning and memory.

Infants, in particular pre-term infants, have a high demand for nutrients including sialic acids due to the rapid brain growth and the development of their immune system at this developmental stage. There are also high levels of sialic acids, particularly Neu5Ac, in human breast milk (∼ 0.5 g/L). In contrast, infant formulas contain low or even negligible amounts of Neu5Ac.

It is therefore necessary to provide sialic acids, particularly Neu5Ac, of a sufficient quality and in sufficient quantity for the supplementation of infant formulas and other nutritional compositions. In this regard, various approaches have been published in the past.

Document EP 1 484 406 A1 describes a process for the production of Neu5Ac using a microorganism which has the ability to synthesize Neu5Ac. After a period of cultivation, the cells were pelleted by centrifugation and stored at -20 °C as so-called "wet cells". For the production of Neu5Ac, the wet cells were thawed and permeabilized for to facilitate an *in vitro* reaction. After the completion of the *in vitro* reaction, the formation of Neu5Ac was evaluated by high-performance liquid chromatography (HPLC).

Document WO 94/29476 A1 discloses an *in vitro* method for the preparation of *N*-acetyl-D-neuraminic acid from *N*-acetyl-D-glucosamine (NAG, GlcNAc). In the preparation, NAG is converted to *N*-acetyl-D-mannosamine (NAM, ManNAc) by base-catalyzed epimerization. Subsequently, NAM reacts with pyruvate in a reaction catalyzed by Neu5Ac-aldolase to yield Neu5Ac. The Neu5Ac-aldolase was prepared from recombinant *E. coli* cells expressing the said Neu5Ac-aldolase. The aldolase enzyme was immobilized by mixing Eupergit-C® beads with a crude extract of said recombinant *E. coli* cells. The conversion of NAM to Neu5Ac was initiated by adding the said immobilized enzyme beads to a mixture of NAM and pyruvate. Neu5Ac was obtained from the reaction mixture in that the enzyme was removed by filtration and the filtrate was mixed with glacial acetic acid and a small quantity of seed to obtain a "wet cake". The wet cake was subjected either to acetone desolvation or rhomboid crystallization.

In an alternative to the previous process, EP 0 578 825 A1 discloses an *in vitro* process for the production of Neu5Ac by treating a mixture of *N*-acetylglucosamine and pyruvic acid with an *N*-acetylneuraminic acid lyase under alkaline conditions. The reaction product was isolated by ion-exchange column chromatography using Dowex 1 (Dow Chemical Company), and isolates were concentrated by crystallization.

US Patent No. US 7,579,175 discloses a process for the production of Neu5Ac by utilizing permeabilized microorganisms. The method comprises the preparation of a mixture containing (i) a culture of a microorganism having *N*-acetylneuraminic acid aldolase activity or *N*-acetylneuraminic acid synthetase activity, or treated matter derived from the culture; (ii) a culture of a microorganism capable of producing pyruvic acid (or treated matter derived from the culture), or a culture of a microorganism capable of producing phosphoenolpyruvic acid (or treated matter derived from the culture); (iii) *N*-acetylmannosamine; and (iv) an energy source which is necessary for the formation of pyruvic acid or phosphoenolpyruvic acid. The mixture is prepared in an aqueous medium containing a chelating agent or surfactant allowing the formation and accumulation of Neu5Ac in the aqueous medium. The reaction products were quantified using a carbohydrate analysis system.

International publication WO 2008/040717 A2 discloses a method for the production of sialic acid comprising the cultivation of a microorganism in a medium. Neu5Ac has been purified from the supernatant (2 liters) of a culture by direct precipitation using glacial acetic acid.

International Publication No. WO 2008/097366 A2 concerns metabolically engineered *E. coli* cells producing sialic acid. Sialic acid was purified from the culture broth by ion-exchange chromatography.

Document CN 2017/10277428 A concerns genetically engineered strain of *Bacillus subtilis* that improves the production of Neu5Ac. The amount of Neu5Ac in fermentation cultures was determined by HPLC.

International Publication No. WO 2012/083329 A1 discloses methods and agents for the production of Neu5Ac by genetically engineered fungal cells of the genus *Trichoderma.* The mycelia of such *Trichoderma* stains were analyzed for the presence of Neu5Ac by HPLC-MS.

Document EP 0 474 410 A2 discloses a method for the production of sialic acid by hydrolyzing delipidated egg yolk. The method comprises the steps of desalting a solution containing sialic acid obtainable by hydrolyzing delipidated egg yolk, adsorbing the sialic acid to an anion-exchange resin and then eluting said sialic acid. The desalting can be achieved by using a reverse osmosis membrane, an electrodialysis membrane or a dialysis membrane. The adsorbing process may comprise passing the desalted hydrolysate through a cation-exchange resin and further passing it through an anion-exchange resin. The final eluate was dried under reduced pressure to obtain a solid.

Japanese Publication JP 08-119986 A describes a method for the purification of sialic acid or an analog thereof comprising the synthesis of Neu5Ac by condensing *N*-acetylmannosamine with pyruvic acid in the presence of sialic aldolase. The solution containing sialic acid or an analog thereof is concentrated using an evaporator. The concentrated solution is mixed with an organic acid containing two or three carbon atoms. Subsequently, the mixture is heated to 50 °C and allowed to stand at 4 °C to precipitate white crystals of sialic acid.

These existing attempts to provide purified sialic acid have involved small-scale demonstrations. There remains a need for industrial-scale sialic acid purification processes, particularly the industrial-scale purification of Neu5Ac to provide the quantity and quality of sialic acids suitable for use in the food industry.

The objective is achieved by providing a method for the purification of Neu5Ac from fermentation broth obtained by cultivating a microorganism that is able to produce Neu5Ac under conditions that are permissive for the microorganism to produce Neu5Ac.

## Summary

In a first aspect, provided is a method for the purification of Neu5Ac from a fermentation broth.

In a second aspect, provided is a preparation of Neu5Ac which has been purified by the method according to the first aspect.

In a third aspect, provided is the use of Neu5Ac according to the second aspect for the manufacture of a nutritional composition.

In a fourth aspect, provided is a nutritional composition containing Neu5Ac according to the second aspect.

## Brief description of the drawings

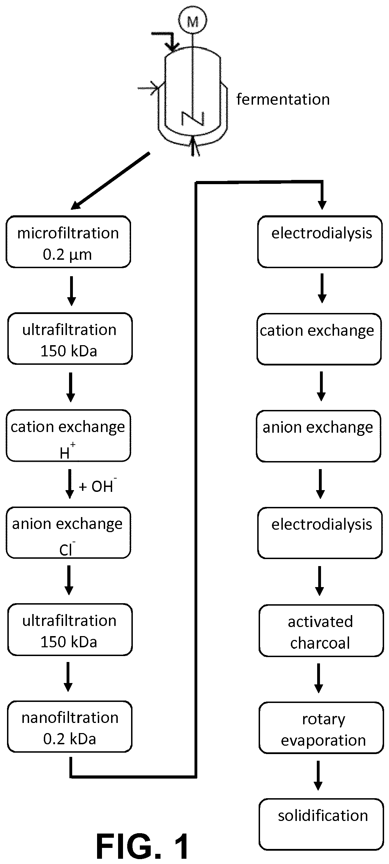

FIG. 1    shows a process chart of a representative embodiment for the purification of Neu5Ac from fermentation broth.

## Detailed description

According to the first aspect, provided is a method for the purification of N-acetylneuraminic acid (Neu5Ac) from a fermentation broth. Said fermentation broth is a liquid medium that is obtained by cultivating a microorganism which is able to produce Neu5Ac therein under conditions that are permissive for the production of Neu5Ac by said microorganism.

The terms "able to" and "capable of" as used herein with respect to the production of Neu5Ac refers to the ability of the microorganism to synthesize Neu5Ac intracellularly and to export the thus synthesized Neu5Ac into the culture medium provided that the microorganism is cultivated under conditions that are permissive for the microorganism to synthesize Neu5Ac.

The term "producing *N*-acetylneuraminic acid" or "producing Neu5Ac" as used herein with respect to a microorganism refers to the intracellular biosynthesis of Neu5Ac by living cells of said microorganism.

The term "export" as used herein with respect to Neu5Ac refers to the transfer of intracellularly-produced Neu5Ac out of the cell and into the fermentation broth in which the Neu5Ac-producing microorganism is cultivated without being bound to any mechanism mediating said transfer of Neu5Ac.

Hence, it is understood that the fermentation of the microorganism that is able to produce Neu5Ac occurs under conditions that are permissive for the microorganism to produce and export Neu5Ac. It is also understood that the fermentation broth obtained by fermentation of said microorganism contains the microorganism, i.e. cells of said microorganism, medium components such as nutrients and ions, Neu5Ac produced by the cells of the microorganism, and by-products such as other metabolites of the microorganism and cellular debris including polypeptides, nucleic acids, and lipids.

The method for the purification of Neu5Ac from a fermentation broth comprises the following steps:

i) removing the biomass from the fermentation broth to obtain a clarified process stream;
ii) removing cations from the clarified process stream by applying at least one cation exchange treatment to the clarified process stream;
iii) removing anionic impurities from the clarified process stream by applying at least one anion exchange treatment to the clarified process stream; and
iv) subjecting the clarified process stream to at least one nanofiltration and/or at least one electrodialysis step.

The method begins with a step to remove the biomass from the fermentation broth (step i). This step is typically the first step in the method for the purification of Neu5Ac from the fermentation broth.

The term "biomass" as used herein refers to the entirety of cells present in the fermentation broth at the end of the fermentation step. The biomass includes the cells of the microorganism that produced Neu5Ac, and descendent cells of the microorganism that may have lost their ability to produce Neu5Ac during the fermentation step as well as any other cells that are unintentionally present in the fermentation broth at the end of the fermentation step. Hence, essentially all cells that are present in the fermentation broth at the end of the fermentation step are separated from the fermentation broth such that the clarified broth, i.e. the process stream, is substantially free of cells.

Suitable methods for removing the biomass from the fermentation broth include centrifugation methods and filtration methods.

In suitable centrifugation methods for removing the biomass from the fermentation broth, the biomass is obtained as a pellet and the supernatant as clarified process stream which is subjected to further treatments.

In an additional and/or alternative embodiment, the biomass is removed from the fermentation broth by means of filtration, preferably by microfiltration. The filtrate becomes the clarified process stream.

Microfiltration is a physical separation process wherein a particle-containing fluid is passed through a medium, said medium comprising either a porous substance containing torturous channels to retain particles (depth filtration) and/or a membrane with a specific pore size allowing the passage of particles/molecules that are smaller than said pore size (membrane filtration or dead-end filtration). The term "microfiltration" as used herein refers to a physical separation process wherein cells are removed from the fermentation broth leaving process stream.

In an additional and/or alternative embodiment, smaller particles and large soluble molecules are removed from the clarified filtrate by means of further filtration, specifically ultrafiltration.

Ultrafiltration is a form of membrane filtration that is not fundamentally different from dead-end microfiltration. In ultrafiltration, forces generated by pressure and concentration gradients lead to the removal of particles and large soluble molecules by passing the liquid containing such particles and large soluble molecules through a semipermeable membrane causing the particles and large soluble molecules to be retained in the so-called retentate, while water and low molecular weight solutes such as Neu5Ac pass through the membrane into the permeate (filtrate).

Membranes for ultrafiltration are defined by their molecular weight cut-off (MWCO) which describes the maximum molecular weight of a soluble molecule that can pass through the membrane in to the permeate. Any particles, as well as molecules larger than the MWCO, are unable to pass through the membrane are remain in the retentate. Ultrafiltration may be applied in cross-flow mode, where the flow of the liquid is parallel to the membrane surface, or in dead-end mode where the flow of the liquid is perpendicular to the membrane surface.

Suitable filters for microfiltration or ultrafiltration for removing the biomass from the fermentation broth include SPIRA-CEL® DS MP005 4333, a module wherein a polyethersulfone membrane having a nominal pore size of 0.05 $\mu$m is spirally wound to provide a compact design and better performance. Suitable membranes for removing the biomass by microfiltration may have a pore size of 0.2 $\mu$m. Alternatively, the biomass may be removed from the fermentation broth by ultrafiltration using a membrane with a MWCO of 500 kDa.

In an additional and/or alternative embodiment, smaller particles and large soluble molecules are removed from the clarified process stream by a cross-flow ultrafiltration, wherein the clarified process stream is subjected to an ultrafiltration step using a filter having a MWCO of 150 kDa, for example FS10-FC FUS1582 (Microdyn-Nadir GmbH, Wiesbaden, DE), a hollow fiber ultrafiltration module using a polyethersulfone membrane (5 m$^2$) with a MWCO of 150,000 Dalton (150 kDa).

The cells that are used to produce Neu5Ac synthesize the desired Neu5Ac intracellularly and secrete it into the fermentation broth. The thus produced Neu5Ac ends up in the fermentation broth which is then subjected to further process steps for purifying the Neu5Ac as described herein after.

Notwithstanding that the process for the purification of Neu5Ac was conceived for the purification of Neu5Ac from fermentation broth, said process may also be used for the purification of Neu5Ac produced by an enzymatic reaction *in vitro*, a so-called *in vitro* biocatalysis reaction or by using a permeabilized whole cell biocatalysis approach. It is understood that the purification of Neu5Ac from the reaction mixture of an *in vitro* biocatalysis reaction does not require removal of biomass from the reaction mixture. The reaction mixture of an *in vitro* biocatalysis reaction thus corresponds to the clarified process stream.

The clarified process stream containing Neu5Ac also contains a substantial amount of undesired impurities including (but not limited to) monovalent ions, divalent ions, amino acids, polypeptides, proteins, organic acids, nucleic acids, monosaccharides and/or oligosaccharides. For the purification of Neu5Ac the method further comprises the step of removing cations from the clarified process stream by applying at least one cation-exchange treatment to the clarified process stream.

Suitable cation-exchange resins for removing positively charged compounds are strongly acidic cation-exchange resins such as (but not limited to) Lewatit® S2568 (H+) (Lanxess AG, Cologne, DE), Dowex® 50WX2 (Merck KGaA, Darmstadt, DE); Amberlite® IR-116 (Japan Organo Co., Ltd.) and Diaion™ SK-102 (Mitsubishi Chemical Industries, Ltd).

During cation exchange, cations within the clarified process stream are replaced by H+. Therefore, the pH of the eluate is acidic and is increased prior to subsequent purification steps, preferably to achieve a neutral or almost neutral pH value (i.e. ≥ pH 6.5) of the eluate, and more preferably by adding NaOH.

The method for the purification of Neu5Ac comprises the step of removing anions from the clarified process stream by applying at least one anion-exchange treatment to the clarified process stream.

In an additional and/or alternative embodiment, the anions are removed from the clarified process stream after the clarified process stream has been subjected to the at least one cation-exchange treatment.

Suitable anion exchange resins are strongly basic (type I) anion exchange resins such as (but not limited to) Lewatit® S6368 A, Lewatit® S4268, Lewatit® S5528, Lewatit® S6368A (Lanxess AG, Cologne, DE), Dowex® AG 1x2 (Mesh 200-400), Dowex ®1x8 (Mesh 100-200), Purolite® Chromalite CGA100x4 (Purolite GmbH, Ratingen, DE), Amberlite® FPA51 (Dow Chemicals, MI, USA). Preferably, the anion exchange resin is present in chloride form.

The method for purifying Neu5Ac comprises a step in which the clarified process stream or eluate from the ion-exchange treatment is subjected to at least one nanofiltration and/or at least one electrodialysis step. Preferably, the eluate from the ion-exchange treatment is subjected to a nanofiltration and/or an electrodialysis, i.e. after cations and/or anions have been removed from the clarified process stream.

Nanofiltration is a membrane filtration method in which the membrane contains nanometer-sized pores. Nanofiltration membranes have pore sizes ranging from 1 to 10 nanometers. The pore size of nanofiltration membranes is smaller than the pore sizes of microfiltration and ultrafiltration membranes, but larger than the pore sizes of membranes used for reverse osmosis.

Membranes for use in nanofiltration are predominantly created from thin polymer films. Materials that are commonly used include polyethylene terephthalate or metals such as aluminum. Pore densities may range from 1 to $10^6$ pores per cm$^2$.

Nanofiltration is used in the method for the purification of Neu5Ac to increase the concentration of Neu5Ac in the solution, i.e. the clarified process stream or an eluate obtained from any steps within the method.

Suitable membranes for nanofiltration include polyamide or polypiperazine, thin-film composite membrane material providing a size exclusion in the range of 150 to 300 Da, for example Dow Filmtec™ NF270 (Dow Chemical Company, USA). Such membranes allow high flux. Additional examples of suitable membranes for nanofiltration include Trisep 4040-XN45-TSF (Microdyn-Nadir GmbH, Wiesbaden, DE), GE4040F30 and GH4040F50 (GE Water & Process Technologies, Ratingen, DE).

Nanofiltration was found to efficiently remove significant amounts of contaminants prior to electrodialysis treatment of the solution containing Neu5Ac. Nanofiltration was also found to be efficient for the removal of low-molecular-weight contaminants after the ultrafiltration step, wherein the removal of low-molecular-weight components is beneficial for concentrating and demineralizing the Neu5Ac-containing solution prior to an ion-exchange treatment. The use of nanofiltration for concentrating the Neu5Ac results in lower energy and processing costs, and better product quality due to reduced thermal exposure.

Electrodialysis combines dialysis and electrolysis, and can be used for the separation and concentration of ions in solutions based on their selective electromigration through a semipermeable membrane.

The basic principle of electrodialysis consists of an electrolytic cell comprising a pair of electrodes submerged into an electrolyte for the conduction of ions, connected to a direct current generator. The electrode connected to the positive pole of the direct current generator is the anode, and the electrode connected to the negative pole is the cathode. The electrolyte solution then supports the current flow, which results from the movement of negative and positive ions towards the anode and cathode, respectively. The membranes used for electrodialysis are essentially sheets of porous ion-exchange resins with negative or positive charge groups, and are therefore described as cationic or anionic membranes, respectively. The ion-exchange membranes usually consist of a polystyrene matrix carrying a suitable functional group (such as sulfonic acid for cationic membranes or a quaternary ammonium group for anionic membranes) cross-linked with divinylbenzene. The electrolyte can be, for example, sodium chloride, sodium acetate, sodium propionate or sulfamic acid. The electrodialysis stack is then assembled in such a way that the anionic and cationic membranes are parallel as in a filter press between two electrode blocks, such that the stream undergoing ion depletion is well separated from the stream undergoing ion enrichment (the two solutions are also referred to as the diluate (undergoing ion depletion) and concentrate (undergoing ion enrichment). The heart of the electrodialysis process is the membrane stack, which consists of several anion-exchange membranes and cation-exchange membranes separated by spacers, installed between two electrodes. By applying a direct electric current, anions and cations will migrate across the membranes towards the electrodes generating a (desalted) diluate stream and a concentrate stream.

By applying an acidic condition during electrodialysis, Neu5Ac can be protonated such that it appears uncharged (protonation of the carbonyl group of the Neu5Ac part of the oligosaccharide). As an alternative, electrodialysis can be performed under neutral conditions using bipolar membranes. In this case the Neu5Ac can be concentrated in a separate electrodialysis concentrate circuit. Thus, Neu5Ac can be enriched during electrodialysis.

The pore size of the ion-exchange membranes for use in electrodialysis is small enough to prevent diffusion of the product from the diluate stream into the concentrate stream, driven by high concentration differences between the two streams. After separation from biomass and/or exchange of cations and/or anions, proteins and in particular recombinant DNA molecules (ranging in size from fragments to entire genomes) must be quantitatively removed from the desired product.

In an additional and/or alternative embodiment, the method for the purification of Neu5Ac further comprises at least one step in which the clarified process stream or the eluate obtained from the ion-exchange steps described above with activated carbon to remove colorants.

Activated carbon, also called activated charcoal, is a form of carbon that has been processed to have small, low-volume pores that increase the surface area available for adsorption. Typically, just one gram of activated carbon has a surface area greater than 3000 m$^2$ as determined by gas adsorption, due to its high degree of microporosity.

Treating the clarified process stream or eluate with activated carbon removes undesired colorants and therefore increases the purity of Neu5Ac.

In an additional and/or alternative embodiment, the method for the purification of Neu5Ac comprises at least one step that increases the concentration of Neu5Ac.

Suitable methods for increasing the concentration of Neu5Ac include nanofiltration and solvent evaporation.

In an additional and/or alternative embodiment of the process according to the invention, the purified solution containing Neu5Ac is filter-sterilized and/or subjected to an endotoxin removal step, preferably by filtration of the purified solution through a 3 kDa filter or 6 kDa filter. The removal of endotoxins is necessary if the Neu5Ac is intended for human consumption.

In additional and/or alternative embodiment of the process, the solution containing the Neu5Ac is concentrated after at least one of the purification steps i) to iv), preferably after purification step iv), using vacuum evaporation (e.g. by using a rotating evaporator or a plate evaporator) or reverse osmosis or nanofiltration (e.g. nanofiltration with a nanofiltration membrane having a size exclusion limit of $\leq$ 20 Å)

a) to a concentration of $\geq$ 100 g/L, preferably $\geq$ 200 g/L, more preferably $\geq$ 300 g/L; and/or
b) at a temperature of < 80°C, preferably < 50°C, more preferably 20 °C to 50 °C, even more preferably 30 °C to 45 °C, most preferably 35 °C to 45 °C (specifically relevant for vacuum evaporation or reverse osmosis); and/or
c) at a temperature of < 80 °C, preferably < 50°C, more preferably 4 °C to 40 °C (specifically relevant for nanofiltration).

The method for the purification of Neu5Ac provides a preparation of Neu5Ac having a purity of $\geq$ 80%, $\geq$85%, $\geq$90%, $\geq$95%.

The term "purity" as used herein refers to chemical purity and specifies the degree to which a substance, i.e. Neu5AC, is undiluted or unmixed with extraneous material. Hence, the chemical purity is an indicator of the relationship between Neu5Ac and by-products/impurities. Chemical purity is expressed as a percentage (%) and is calculated using the following formula:

$$\text{Percent Purity} = 100 \times \frac{\text{Mass of desired compound in sample}}{\text{Total mass of sample}}$$

The purity of a preparation of Neu5Ac may be determined by any suitable method known to the skilled artisan, for example by using HPLC and calculating the ratio of the area underneath the peaks representing the amount of Neu5Ac to the sum of areas underneath the peaks representing Neu5Ac and other compounds than Neu5Ac in the same chromatogram.

The method provides a preparation of Neu5Ac of sufficient purity to allow its use in food or feed applications. The method for the purification of Neu5Ac is also advantageous in that the preparation of Neu5Ac is free of recombinant DNA and recombinant proteins derived from recombinant microbial fermentation strains which may be used for the production of Neu5Ac. Furthermore, the method is cost efficient and easy to scale up such that it suitable for the multi-ton-scale manufacturing of Neu5Ac by fermentation.

In an additional and/or alternative embodiment, the process further includes a step for the removal of solvent or moisture from the Neu5Ac such that either a solution of Neu5Ac is provided which includes a high concentration of Neu5Ac, or such that a solid preparation of Neu5Ac is obtained.

In additional and/or alternative embodiment, the method further includes a step to obtain Neu5Ac in solid form. Suitable methods for obtaining Neu5Ac in solid form include precipitation of Neu5Ac, crystallization of Neu5Ac, evaporation of solvent such as lyophyllization (freeze-drying) or spray-drying.

Precipitation is the formation of a solid by removing a solute from a solution. When the reaction occurs in a liquid solution, the solid formed is called the 'precipitate'. Precipitation may occur if the concentration of a compound exceeds its solubility (such as when mixing solvents or altering the temperature or pressure).

The crystallization of Neu5Ac from an aqueous solution is achieved by adding an excess of an alcohol such as ethanol or of an organic acid such as glacial acetic acid. The crystallization of Neu5Ac is a process by which Neu5Ac precipitates from the solvent in solid form, wherein the Neu5Ac molecules are present in a highly organized structure

The lyophilization of Neu5Ac is a process in which a Neu5Ac-containing aqueous solution is frozen and then the pressure is reduced such that the frozen water sublimes directly from the solid phase to the gas phase - this usually produces a hygroscopic Neu5Ac powder.

In an alternative embodiment, the solvent is removed from the liquid preparation of Neu5Ac by spray-drying.

According to the second aspect, provided are preparations of Neu5Ac, wherein the Neu5Ac was produced by microbial fermentation and purified from the fermentation broth as disclosed herein before.

The preparation of Neu5Ac may be in liquid form as a solution containing Neu5Ac in a solvent, preferably in water. In an alternative embodiment, the preparation of the Neu5Ac is present in solid form, preferably in the form of a powder. The powder contains the Neu5Ac in the form of particles, wherein the Neu5Ac is present in as amorphous particles or as crystalline particles. More preferably, the powder containing Neu5Ac has a water content of less than 10%.

The preparation of Neu5Ac obtained by a process as described herein before preferably comprises the desired Neu5Ac with a purity of ≥ 80% by weight.

According to the third aspect, provided is the use of Neu5Ac obtained by a method as described herein before for the manufacture of a nutritional composition, preferably for the preparation of an infant formula.

The method of purifying Neu5Ac provides preparations of Neu5Ac wherein the Neu5Ac has a purity sufficient for human consumption. Hence, said Neu5Ac may be used for human consumption and in compositions intended for human consumption, such as nutritional compositions.

Thus, according to the fourth aspect, provided are nutritional compositions containing Neu5Ac which has been produced by a method as disclosed herein before.

The nutritional compositions further contain micronutrients and/or macronutrients such as proteins, carbohydrates, fats, fatty acids, preferably polyunsaturated fatty acids (PUFAs), vitamins and minerals.

In an additional and/or alternative embodiment, the nutritional composition contains at least one human milk oligosaccharide (HMO). The at least one HMO may be a neutral HMO, preferably selected from the group consisting of 2'-fucosyllactose (2'-FL), 3-fucosyllactose (3-FL), lacto-*N*-tetraose (L*N*T), lacto-*N-neo*tetraose (L*N*nT), and lacto-*N*-fucopentaose I (L*N*FPI). In an additional and/or alternative embodiment, the at least one HMO may be a sialylated HMO, preferably selected from the group consisting of 3'-sialyllactose (3'-SL), 6'-sialyllactose (6'-SL), sialyllacto-*N*-tetraose (LST)-a, LST-b, LST-c and disialyllacto-*N*-tetraose (DSLNT).

In an additional and/or alternative embodiment, the nutritional composition includes a mixture consisting essentially of Neu5Ac, 2'-FL, 3-FL, L*N*T, L*N*nT, L*N*FPI, 3'-SL, 6'-SL and L-fucose. A composition comprising preferred amounts of each of said compounds is provided in Table 1.

Table 1: Composition of a representative mixture containing Neu5AC suitable for infant formulas.

| Compound | Proportion in mix (percentage by weight) | Final concentration in infant formula (g/L) |
|----------|------------------------------------------|---------------------------------------------|
| 2'-FL | 34 | 2.5 |
| 3-FL | 11 | 0.8 |
| L*N*T | 20 | 1.5 |

(continued)

| Compound | Proportion in mix (percentage by weight) | Final concentration in infant formula (g/L) |
| --- | --- | --- |
| LNnT | 2 | 0.15 |
| LNFPI | 13 | 1.0 |
| 3'-SL | 3 | 0.2 |
| 6'-SL | 4 | 0.3 |
| Neu5Ac | 8 | 0.6 |
| L-Fucose | 5 | 0.4 |
| **Total** | **100** | **7.45** |

The composition according to the second column in Table 1 is of particular advantage for supplementing infant formula such that the final infant formula for direct consumption may contain the compounds of the mixture in concentrations as specified in the third column of Table 1.

In an additional embodiment, the nutritional composition is selected from the group consisting of medicinal formulations, infant formulas, dairy drinks and dietary supplements.

As a medicinal formulation, the nutritional composition may be used to improve cognitive performance, especially for improving attention, learning and/or memory.

As an infant formula, the nutritional composition meets the compositional requirements set forth in Regulation (EU) 2016/127. Representative compositions of infant formulas are specified in Tables 2 and 3.

Table 2: Components of a representative infant formula.

| Infant formula: | Skimmed milk |
| --- | --- |
| | Vegetable oils (palm oil, rapeseed oil, sunflower oil) |
| | Human milk oligosaccharides |
| | Neu5Ac |
| | Skimmed milk powder |
| | Oil of *Mortierella alpine* |
| | Fish oil |
| | Calcium carbonate |
| | Potassium chloride |
| | Vitamin C |
| | Sodium chloride |
| | Vitamin E |
| | Iron acetate |
| | Zinc sulfate |
| | Niacin |
| | Calcium-D-panthothenate |
| | Copper sulfate |
| | Vitamin A |
| | Vitamin B1 |
| | Vitamin B6 |
| | Magnesium sulfate |
| | Potassium iodate |
| | Folic acid |
| | Vitamin K |
| | Sodium selenite |
| | Vitamin D |

Table 3: Composition of a representative infant formula. The final concentration is based on a preparation of 13.5 g of the powder in 90 ml of water.

|  |  | per 100 g powder | per 100 ml infant formula |
|---|---|---|---|
| Energy | kJ | 2094-2145 | 283 |
|  | kcal | 500-512 | 67-68 |
| Fats, hereof: | g | 24.2-26.2 | 3.3-3.5 |
| saturated fatty acids | g | 8.7-9.4 | 1.2-1.3 |
| monounsaturated fatty acids | g | 10.4 | 1.4 |
| polyunsaturated fatty acids | g | 5.5-5.9 | 0.7-0.8 |
| Carbohydrates hereof: | g | 56-58 | 7.4-7.9 |
|  |  |  |  |
| Sugars | g | 44-56 | 6-7.4 |
| hereof: |  |  |  |
| Lactose | g | 44-56 | 6-7.4 |
| Neu5Ac | mg | 440 | 60 |
| L-fucose | mg | 300 | 40 |
| HMOs Hereof | g | 4.22-4.81 | 0.57-0.65 |
| 2'-FL | g | 1.85-2.22 | 0.25-0.30 |
| 3-FL | mg | 555.56-592.6 | 75-80 |
| L$N$T | g | 1.11 | 0.15 |
| L$N$nT | mg | 0-111.11 | 0-15 |
| L$N$PF-I | mg | 0-740.74 | 0-100 |
| 3'-SL | mg | 148.15-170.37 | 20-23 |
| 6'-SL | mg | 207.4-222.22 | 28-30 |
| Protein | g | 11.11-11.85 | 1.5-1.6 |
| Salt | g | 0.47-0.59 | 0.06-0.08 |
| Vitamins |  |  |  |
| Vitamin A | μg | 357-358 | 47.3-48.2 |
| Vitamin D | μg | 7.8 | 1.05 |
| Vitamin E | mg | 8.15 | 1.1 |
| Vitamin K | μg | 43.7-44.4 | 5.9-6.0 |
| Vitamin C | mg | 115-118 | 15-16 |
| Vitamin B1 | mg | 0.51-0.60 | 0.068-0.079 |
| Vitamin B2 | mg | 1.3-1.7 | 0.18-0.23 |
| Niacin | mg | 3.63 | 0.49 |
| Vitamin B6 | μg | 526-600 | 71-81 |
| Folic acid | μg | 160-164 | 21.6-21.7 |
| Vitamin B12 | μg | 1.7-1.9 | 0.23-0.25 |
| Biotin | μg | 22-30 | 3.0-3.9 |

(continued)

|  |  | per 100 g powder | per 100 ml infant formula |
|---|---|---|---|
| Panthothenic acid | mg | 4.6-5.4 | 0.62-0.72 |
| Minerals |  |  |  |
| Sodium | mg | 187-236 | 25.3-31.2 |
| Potassium | mg | 673-675 | 88.8-91.2 |
| Chloride | mg | 327-333 | 43.1-44.9 |
| Calcium | mg | 460-504 | 62.1-66.5 |
| Phosphorous | mg | 335-352 | 45.2-46.5 |
| Magnesium | mg | 49.3-56.3 | 6.66-7.43 |
| Iron | mg | 4.15 | 0.56 |
| Zinc | mg | 3.7-3.8 | 0.49-0.51 |
| Copper | μg | 274 | 37 |
| Manganese | μg | 96.3 | 13 |
| Fluoride | μg | 30.4-32.6 | 4.1-4.4 |
| Selenium | μg | 11.1-12.3 | 1.5-1.6 |
| Iodine | μg | 101.5-103.7 | 13.7-14 |

In an additional and/or alternative embodiment, the nutritional composition also contains microorganisms, preferably probiotic microorganisms. For infant food applications, the preferred microorganisms are derived from or can be found in the microbiome of a healthy human. Preferably, but with no limitations, the microorganisms are selected from the genera *Bifidobacterium*, *Lactobacillus*, *Enterococcus*, *Streptococcus*, *Staphylococcus*, *Peptostreptococcus*, *Leuconostoc*, *Clostridium*, *Eubacterium*, *Veilonella*, *Fusobacterium*, *Bacterioides*, *Prevotella*, *Escherichia*, *Propionibacterium* and *Saccharomyces.* In an additional and/or alternative embodiment, the microorganism is selected from the group consisting of *Bifidobacterium adolescentis, B. animalis, B. bifidum, B. breve, B. infantis, B. lactis, B. longum; Enterococcus faecium; Escherichia coli; Klyveromyces marxianus; Lactobacillus acidophilus, L. bulgaricus, L. casei, L. crispatus, L. fermentum, L. gasseri, L. helveticus, L. johnsonii, L. paracasei, L. plantarum, L. reuteri, L. rhamnosus, L. salivarius, L. sakei; Lactococcus lactis* (including but not limited to the subspecies *lactis, cremoris* and *diacetylactis*); *Leuconostoc mesenteroides* (including but not limited to subspecies *mesenteroides*); *Pedicoccus acidilactici, P. pentosaceus; Propionibacterium acidipropionici, P. freudenreichii* ssp. *shermanii*; *Staphylococcus carnosus*; and *Streptococcus thermophilus.*

In addition to the combination of Neu5Ac with living organisms, Neu5Ac can be also used in combination with dead cultures. In the field of probiotics, killed cultures are sometimes used (e.g. tyndalized bacteria). These killed cultures may provide proteins, peptides, oligosaccharides, cell outer wall fragments and natural products, leading to the short-term stimulation of the immune system.

The combination of Neu5Ac and probiotic microorganisms in the nutritional composition, especially in the presence of HMOs, is particularly advantageous in that it also promotes the establishment of a healthy gut microbiome.

In an additional and/or alternative embodiment, the nutritional composition also includes prebiotics such as galacto-oligosaccharides (GOS), fructo-oligosaccharides (FOS), inulin or combinations thereof.

The nutritional composition may be present in liquid form or in solid form including, but not limited to, powders, granules, flakes and pellets.

Provided is also a nutritional composition comprising Neu5Ac and at least six HMOs, wherein at least five of these HMOs are selected form the group consisting of 2'-FL, 3-FL, L*N*T, L*N*nT, L*N*FPI, 3'-SL and 6'-SL.

In an additional embodiment, the nutritional composition further contains L-fucose.

In an additional and/or alternative embodiment, a nutritional composition as disclosed herein before contains at least one sialylated HMO and at least one neutral HMO and a probiotic microorganism.

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", as used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification do not necessarily refer always to the same embodiment. Furthermore, the particular features, structures or characteristics may be combined in any suitable manner, as would be apparent to one of ordinary skill in the art from this disclosure, in one or more embodiments.

Similarly, it should be appreciated that in the description of representative embodiments of the invention, various features of the invention are sometimes grouped together in a single embodiment, figure, or description thereof for the purpose of streamlining the disclosure and facilitating the understanding of one or more of the various inventive aspects. This method of disclosure is not to be interpreted as reflecting an intention that the claimed invention requires more features than are expressly listed in each claim. Rather, as the following claims reflect, inventive aspects may require fewer than all the features of any foregoing disclosed embodiment. Thus, the claims following the detailed description are hereby expressly incorporated into this detailed description, with each claim standing on its own as a separate embodiment of this invention.

Furthermore, whereas some embodiments described herein include some but not other features included in other embodiments, combinations of features of different embodiments are meant to be within the scope of the invention, and form different embodiments, as would be understood by those familiar with the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order to facilitate the understanding of the description and drawings.

The invention will now be described by a detailed description of several embodiments of the invention. It is clear that other embodiments of the invention can be configured according to the knowledge of persons skilled in the art without departing from the true spirit or technical merit of the invention, the invention being limited only by the terms of the appended claims.

**Example 1: Purification of *N*-acetylneuraminic acid from fermentation broth**

A fed-batch fermentation using recombinant *E. coli* that can produce *N*-acetylneuraminic acid (Neu5Ac) was performed in that bacterial cells of said strain were grown in a defined medium (fermentation broth).

Following the fermentation run, the biomass was separated from the fermentation broth by microfiltration and ultrafiltration using sequentially a winding module filter (polyethersulfone membrane having a nominal pore size of 0.05 $\mu$m; CUT membrane technology, Erkrath, Germany), and a cross-flow filter (150 kDa MWCO; Microdyn-Nadir, Wiesbaden, Germany). This yielded ~1 m$^3$ of clarified filtrate containing > 19 g·L$^{-1}$ *N*-acetylneuraminic acid (Neu5Ac).

The filtrate was then deionized by ion-exchange chromatography. First, cationic contaminants were removed on a strong cationic exchanger with a bed volume of 200 L (Lewatit® S 2568 (Lanxess AG, Cologne, Germany) in H$^+$ form. The acidic eluate (pH ~1.5) was then neutralized to pH 7.0 with NaOH. Second, anionic contaminants were removed from the solution using the strong anionic exchanger Lewatit® S 6368 A (Lanxess AG, Cologne, Germany) in the chloride form. The anion exchanger also had a bed volume of 200 L. A further cross-flow filtration step (150 kDa cut-off; Microdyn-Nadir GmbH, Wiesbaden, Germany) was used to remove any precipitates that formed due to the acidification of the solution. The solution containing Neu5Ac was then concentrated to -25% of the original volume by nanofiltration using a Dow Filmtec® NF270-4040 (INAQUA Vertriebsgesellschaft mbH, Mönchengladbach, Germany). The Neu5Ac solution was then further concentrated to ≥ 400 g/L on a rotary evaporator. Specific crystallization of the product was achieved by adding a 10-fold excess of glacial acetic acid at 5°C for 12-60 h. The solid fraction was washed with ethanol and dried at 40°C. The dry crystallized product was further purified by dissolving in 2 L of water per kg before passing through activated charcoal (CAS-No: 7440-44-0, Carl Roth GmbH & Co. KG, Karlsruhe, Germany). The purified solution was separated from the charcoal and concentrated by evaporation at 50°C until only solid residue remained. This solid material was mixed with 99% ethanol and incubated at 4°C for at least for 16 h. Afterwards, the solid fraction was dried at 40°C. A crystalline, white product was obtained with a purity of more than 95% as determined by measuring the area under the curve of a chromatogram produced by HPLC using a Rezex ROA-organic acid H$^+$ column (Phenomenex, Aschaffenburg, Germany).

A fed-batch fermentation using recombinant *E. coli* (strain #2152) that can produce *N*-acetylneuraminic acid (Neu5Ac) was performed in that bacterial cells of said strain were grown in a defined medium (fermentation broth).

The bacterial cells were removed from the fermentation broth by two-step filtration. In the first step a filter with a MWCO of 500 kDa was used. In the second filtration step a cross-flow filter with a MWCO of 150 kDa was used. Removing the bacterial cells from the fermentation broth produced ~1 m$^3$ of clarified filtrate containing about 19 g/L Neu5Ac.

The clarified filtrate was passed over a cation exchanger (Lewatit® S 2568) in order to remove positively charged compounds. Lewatit® S 2568 (Lanxess Deutschland GmbH, Germany) is a food-grade, macroporous, monodisperse, strongly acidic cation-exchange resin based on a styrene-divinylbenzene copolymer.

The eluate was then passed over an anion exchanger (Lewatit® S 6368 A). Lewatit® *S 6368* A (Lanxess Deutschland GmbH, Germany) is a food-grade, macroporous, monodisperse, strongly basic (type I) anion-exchange resin based on a styrene-divinylbenzene copolymer. The anion-exchange resin was present in chloride (Cl$^-$) form.

The resulting solution was subjected to a cross-flow filtration using a filter having a MWCO of 150 kDa to remove precipitates that might have formed during the previous purification steps.

The concentration of constituents of the thus resulting solution was increased by nanofiltration using a FILMTEC™ NF270-4040 membrane element (DOW). By the end of the nanofiltration step, the concentration of Neu5Ac in the solution had increased 5.3-fold as compared to the concentration of Neu5Ac in the solution prior to nanofiltration.

The solution was then subjected first to a first electrodialysis in an acidic environment (pH 1.8, adjusted with HCl), subsequently to a cation-exchange treatment using a PC_SK cation-exchange membrane (PCA-Polymerchemie Altmeier GmbH, Heusweiler, DE), and an anion-exchange treatment using a PC Acid 60 membrane (PCA-Polymerchemie Altmeier GmbH, Heusweiler, DE) prior to a further electrodialysis.

The thus obtained concentrated Neu5Ac solution was then treated with activated charcoal (CAS: 7440-44-0, Carl Roth, Karlsruhe, Germany) to remove colorants such as Maillard reaction products and aldol reaction products. The diluted solution was concentrated on a rotary evaporator to a concentration of -300 g/L.

The purified solution was separated from the charcoal and concentrated by evaporation at 50°C until only solid residue remained. This solid material was mixed with 99% ethanol and incubated at 4°C for at least for 16 h. Afterwards, the solid fraction was dried at 40°C. A crystalline, white product was obtained with a purity of more than 95% as determined by measuring the area under the curve of a chromatogram produced by HPLC using a Rezex ROA-organic acid H$^+$ column (Phenomenex, Aschaffenburg, Germany).


**Example 2: Purification of *N*-acetylneuraminic acid from fermentation broth**

A fed-batch fermentation using recombinant *E. coli* that can produce *N*-acetylneuraminic acid (Neu5Ac) was performed in that bacterial cells of said strain were grown in a defined medium (fermentation broth).

The bacterial cells were removed from the fermentation broth by two-step filtration. In the first step a membrane having a nominal pore size of 0.2 μm was used. In the second filtration step a filter with a MWCO of 150 kDa was used. Removing the bacterial cells from the fermentation broth produced ~1 m$^3$ of clarified filtrate containing about 19 g/L Neu5Ac.

The clarified filtrate was passed over a cation exchanger (Lewatit® S 2568) in order to remove positively charged compounds. Lewatit® S 2568 (Lanxess Deutschland GmbH, Germany) is a food-grade, macroporous, monodisperse, strongly acidic cation-exchange resin based on a styrene-divinylbenzene copolymer.

The eluate was then passed over an anion exchanger (Lewatit® S 6368 A). Lewatit® *S 6368* A (Lanxess Deutschland GmbH, Germany) is a food-grade, macroporous, monodisperse, strongly basic (type I) anion-exchange resin based on a styrene-divinylbenzene copolymer. The anion-exchange resin was present in chloride (Cl$^-$) form.

The resulting solution was subjected to a cross-flow filtration using a filter having a MWCO of 150 kDa to remove precipitates that might have formed during the previous purification steps.

The concentration of constituents of the thus resulting solution was increased by nanofiltration using a FILMTEC™ NF270-4040 membrane element (DOW). By the end of the nanofiltration step, the concentration of Neu5Ac in the solution had increased 5.3-fold as compared to the concentration of Neu5Ac in the solution prior to nanofiltration.

The solution was then subjected first to a first electrodialysis in an acidic environment (pH 1.8, adjusted with

HCl), subsequently to a cation-exchange treatment using a PC_SK cation-exchange membrane (PCA-Polymerchemie Altmeier GmbH, Heusweiler, DE), and an anion-exchange treatment using a PC Acid 60 membrane (PCA-Polymerchemie Altmeier GmbH, Heusweiler, DE) prior to a further electrodialysis.

The thus obtained concentrated Neu5Ac solution was then treated with activated charcoal (CAS:7440-44-0, Carl Roth, Karlsruhe, Germany) to remove colorants such as Maillard reaction products and aldol reaction products. The diluted solution was concentrated on a rotary evaporator to a concentration of ~300 g/L.

The purified solution was separated from the charcoal and concentrated by evaporation at 50°C until only solid residue remained. This solid material was mixed with 99% ethanol and incubated at 4°C for at least for 16 h. Afterwards, the solid fraction was dried at 40°C. The process is schematically shown in Fig. 1. A crystalline, white product was obtained with a purity of more than 95% as determined by measuring the area under the curve of a chromatogram produced by HPLC using a Rezex ROA-organic acid H$^+$ column (Phenomenex, Aschaffenburg, Germany).

## Claims

1. A method for the purification of *N*-acetylneuraminic acid (Neu5Ac) from a fermentation broth obtained by microbial fermentation of a microorganism which is capable of producing Neu5Ac and secreting said Neu5Ac into the fermentation broth, the fermentation broth containing Neu5Ac, the microorganisms, other medium components and contaminants, the method comprising the following steps:

   i) separating the microorganism(s) from the fermentation broth to obtain a clarified process stream;
   ii) removing cations from the from the clarified process stream by at least one cation exchange treatment of the clarified process stream;
   iii) removing anionic impurities from the cell- clarified process stream by at least one anion exchange treatment of the clarified process stream; and
   iv) subjecting the clarified process stream to at least one nanofiltration and/or at least one electrodialysis step for concentration and further purification.

2. The method according to claim 1, wherein at least one of the steps ii) to iv) is performed at least twice in the course of the purification of the Neu5Ac.

3. The method according to claim 1 or 2, wherein separating the microorganisms from the fermentation broth is performed by microfiltration, preferably in that the microfiltration involves the use of a depth filter and/or a membrane with a nominal cut-off of 0.2 μm or 0.05 μm.

4. The method according to any one of claims 1 to 3, the method further comprises the step of the removal of particulates and large soluble contaminants by

   a) dead-end ultrafiltration, preferably in that the ultrafiltration uses a membrane having a molecular weight cut-off of 500 kDa, and/or
   b) cross-flow ultrafiltration, preferably in that the cross-flow filtration uses a membrane having a molecular weight cut-off of 150 kDa.

5. The method according to any one of claims 1 to 4, wherein the method further comprises at least one step of treating the clarified process stream with activated carbon.

6. The method according to any one of claims 1 to 5, wherein the method is performed as a batch process, a fed-batch process or as a continuous process.

7. The method according to any one of claims 1 to 6, wherein the method further comprises a step to obtain Neu5Ac in solid form, preferably by means of crystallization, precipitation, lyophyllization or spray-drying.

8. A preparation of Neu5Ac, wherein the Neu5Ac has been purified by the method according to any one of claims 1 to 7.

9. Use of Neu5Ac obtained by the method according to any one of claims 1 to 7 for the manufacture of a nutritional composition.

10. A nutritional composition containing Neu5Ac, wherein said Neu5Ac has been produced by microbial fermentation and purified by a method according to any one of claims 1 to 7.

11. The nutritional composition according to claim 10, wherein said nutritional composition is a medicinal formulation, a dietary supplement, a dairy drink or an infant formula.

12. The nutritional composition according to claim 10 or 11, wherein said nutritional composition further contains at least one HMO, preferably at least one neutral HMO and/or at least one sialylated HMO.

13. The nutritional composition according to claim 12, wherein the at least one neutral HMO is selected from the group consisting of 2'-fucosyllactose, 3-fucosyllactose, lacto-N-tetraose, lacto-*N-neo*tetraose and lacto-N-fucopentaose I.

14. The nutritional composition according to claim 12 or 13, wherein the at least one sialylated HMO is selected from the group consisting of 3'-sialyllactose, 6'-sialyllactose, sialyllacto-*N*-tetraose (LST)-a, LST-b, LST-c and disialyllacto-N-tetraose.

15. The nutritional composition according to any one of claims 10 to 14, wherein the nutritional composition contains at least one probiotic microorganism.

FIG. 1

Europäisches
Patentamt

European
Patent Office

Office européen
des brevets

## EUROPEAN SEARCH REPORT

Application Number

EP 17 20 2833

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2008/040717 A2 (CENTRE NATIONAL DE LA RECHERCHE SCIENTIFIQUE (CNRS)) 10 April 2008 (2008-04-10) * page 16; example 1 * * pages 17-19; examples 4-6 * ----- | 1-7 | INV. C12P19/26 C07H13/02 C07H1/06 A23L33/125 |
| Y,D | EP 0 474 410 A2 (TAIYO KAGAKU CO., LTD.) 11 March 1992 (1992-03-11) * page 3, lines 1-12 * * page 3, line 20 - page 4, line 11 * * page 10; claims 4-6 * ----- | 1-7 | |
| A | DATABASE WPI Week 201570 Thomson Scientific, London, GB; AN 2015-440430 XP002781294, & CN 104 628 794 A (WUHAN ZHONGKE GUANGGU GREEN BIOLOGICAL) 20 May 2015 (2015-05-20) * abstract * ----- | 1-7 | |
| A | DATABASE WPI Week 199629 Thomson Scientific, London, GB; AN 1996-283501 XP002781295, & JP H08 119986 A (NISSIN SHOKUHIN KAISHA LTD) 14 May 1996 (1996-05-14) * abstract * ----- | 1-7 | TECHNICAL FIELDS SEARCHED (IPC) C12P C07H A23L |

~~The present search report has been drawn up for all claims~~

| Place of search | Date of completion of the search | Examiner |
|---|---|---|
| Berlin | 23 May 2018 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 17 20 2833

The Search Division considers that the present European patent application does not comply with the requirements of unity of invention and relates to several inventions or groups of inventions, namely:

1. claims: 1-7

       method for the purification of N-acetylneuraminic acid (Neu5Ac) from a fermentation broth obtained by microbial fermentation of a microorganism which is capable of producing Neu5Ac and secreting said Neu5Ac into the fermentation broth, the fermentation broth containing Neu5Ac, the microorganisms, other medium components and contaminants, the method comprising the following steps:
       i) separating the microorganism(s) from the fermentation broth to obtain a clarified process stream,
       ii) removing cations from the from the clarified process stream by at least one cation exchange treatment of the clarified process stream,
       iii) removing anionic impurities from the cell- clarified process stream by at least one anion exchange treatment of the clarified process stream, and
       iv) subjecting the clarified process stream to at least one nanofiltration and/or at least one electrodialysis step for concentration and further purification
                      ---

2. claim: 8

       preparation of Neu5Ac, wherein the Neu5Ac has been purified by the method according to any one of claims 1 to 7
                      ---

3. claims: 9-15

       use of Neu5Ac obtained by the method according to any one of claims 1 to 7 for the manufacture of a nutritional composition,
       nutritional composition containing Neu5Ac, wherein said Neu5Ac has been produced by microbial fermentation and purified by a method according to any one of claims 1 to 7
                      ---

## ANNEX TO THE EUROPEAN SEARCH REPORT
## ON EUROPEAN PATENT APPLICATION NO.

EP 17 20 2833

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2018

| Patent document cited in search report | | Publication date | Patent family member(s) | | Publication date |
|---|---|---|---|---|---|
| WO 2008040717 | A2 | 10-04-2008 | AT | 503836 T | 15-04-2011 |
| | | | CA | 2665332 A1 | 10-04-2008 |
| | | | EP | 2076601 A2 | 08-07-2009 |
| | | | JP | 2010505403 A | 25-02-2010 |
| | | | US | 2011165626 A1 | 07-07-2011 |
| | | | WO | 2008040717 A2 | 10-04-2008 |
| EP 0474410 | A2 | 11-03-1992 | CA | 2050125 A1 | 05-03-1992 |
| | | | DE | 69122936 D1 | 05-12-1996 |
| | | | DE | 69122936 T2 | 06-03-1997 |
| | | | EP | 0474410 A2 | 11-03-1992 |
| | | | US | 5233033 A | 03-08-1993 |
| CN 104628794 | A | 20-05-2015 | NONE | | |
| JP H08119986 | A | 14-05-1996 | JP | 2781140 B2 | 30-07-1998 |
| | | | JP | H08119986 A | 14-05-1996 |

EPO FORM P0459

For more details about this annex : see Official Journal of the European Patent Office, No. 12/82

**EP 3 486 326 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1484406 A1
- WO 9429476 A1
- EP 0578825 A1
- US 7579175 B
- WO 2008040717 A2
- WO 2008097366 A2
- CN 201710277428 A
- WO 2012083329 A1
- EP 0474410 A2
- JP 8119986 A